# FASCICULE DE BREVET EUROPEEN

(11) **EP 3 171 940 B1**
(45) Date de publication et mention de la délivrance du brevet: **24.10.2018**
(21) Numéro de dépôt: 15753730.9
(22) Date de dépôt: 24.07.2015
(51) Int. Cl.: A61N 5/06

(54) **DISPOSITIF TEXTILE MONOPATIENT POUR LE TRAITEMENT PAR PHOTOTHÉRAPIE ET INSTALLATION COMPRENANT UN TEL DISPOSITIF TEXTILE MONOPATIENT**
TEXTILE EINZELPATIENT-VORRICHTUNG FÜR FOTOTHERAPIE UND EINHEIT MIT DER TEXTILEN EINZELPATIENT-VORRICHTUNG
SINGLE-PATIENT TEXTILE DEVICE FOR PHOTOTHERAPY TREATMENT AND UNIT COMPRISING SUCH A SINGLE-PATIENT TEXTILE DEVICE

(30) Priorité: 25.07.2014 US 201462028817 P
(43) Date de publication de la demande: 31.05.2017
(73) Titulaire: Neomedlight, 69100 Villeurbanne (FR)
(72) Inventeur: HUYGHE, Clarisse, 69001 Lyon (FR); PERUCHON, Laure, 69100 Villeurbanne (FR); BROCHIER,Cédric, 69007 Lyon (FR); SAINT GIRONS, Pierre, 69003 Lyon (FR)
(74) Mandataire: Cabinet Laurent & Charras
(86) Numéro de dépôt international: PCT/FR2015/052058
(87) Numéro de publication internationale: WO 2016/012732

(56) Documents cités:
- WO-A1-2009/139877
- WO-A1-2013/068518
- WO-A2-2007/091188
- US-A1- 2004 143 307

## Description

### DOMAINE TECHNIQUE

L'invention se rapporte à l'industrie des traitements par photothérapie.

L'invention concerne plus précisément un dispositif souple de traitement par photothérapie permettant de soigner les patients atteints notamment de l'ictère du nourrisson et de la maladie rare de Crigler-Najjar.

### ETAT DE LA TECHNIQUE

L'ictère du nourrisson, ou encore la maladie rare de Crigler-Najjar sont des maladies liées à un taux de bilirubine libre trop élevé. La bilirubine libre s'accumule dans l'organisme et entraîne une jaunisse (coloration jaune de la peau et des yeux) pouvant causer des dommages neurologiques lorsque le taux de bilirubine est très élevé. La jaunisse est très souvent observée chez le nouveau-né bien que pouvant être également présente chez l'adulte. La maladie de Crigler-Najjar est quant à elle une maladie génétique rare du foie. Le traitement symptomatique de la maladie Crigler-Najjar consiste en une photothérapie quotidienne de 10 à 12 heures, tandis que pour l'ictère du nourrisson le traitement peut s'étendre jusqu'à 24h.

On observe ces dernières années un intérêt particulier du monde médical pour le développement et l'optimisation des traitements par photothérapie efficaces contre les maladies dues à un taux de bilirubine anormalement élevé. Le traitement par photothérapie est le traitement le plus utilisé dans les hôpitaux car beaucoup moins lourd à mettre en place que les autres traitements pouvant être appliqués, soit l'exsanguino-transfusion (technique permettant le remplacement d'une grande partie du sang d'un individu) pour l'ictère du nourrisson ou la greffe de foie pour les malades Crigler-najjar.

De façon générale, les dispositifs de traitement par photothérapie de la jaunisse sont bien connus de l'homme du métier. Des dispositifs divers et variés sont actuellement présents sur le marché. On note l'émergence de deux catégories de dispositifs à savoir les dispositifs rigides, formés d'une coque, et d'autres plus souples qui sont des dispositifs textiles lumineux.

Ce sont les dispositifs textiles souples, de par leur attractivité à la fois en termes de coût et de confort, qui ont fait l'objet ces dernières années des avancées les plus intéressantes et qui ont de ce fait su conquérir les parts de marché les plus importantes.

De manière générale, un dispositif de traitement par photothérapie dit souple, est composé d'une source lumineuse dans le domaine du visible émettant à des longueurs d'ondes qui correspondent à la lumière bleue. La lumière bleue absorbée par la bilirubine, permet de convertir la bilirubine libre en produits de dégradation hydrosolubles facilement éliminés dans les urines et non néfastes pour l'homme. Cette source lumineuse est raccordée par un connecteur optique à un câble. Ce câble contient une pluralité de fibres optiques qui conduisent la lumière jusqu'au patient. Bien souvent, les fibres optiques émergent du câble sous la forme d'un « pad » qui est destiné à être inséré dans un logement. Ces dispositifs, souples, font l'objet de développements récents dans le but d'améliorer et d'optimiser l'efficacité du traitement par photothérapie.

On connait notamment des dispositifs tels que décrit dans le document WO 2004/064924. Ces dispositifs comprennent une zone dite matelassée en partie basse du dispositif permettant au patient de venir s'y appuyer. En effet, au cours du traitement, le patient est amené à bouger. En pratique, on observe que les nourrissons ont tendance à glisser vers la partie basse du dispositif. Ces dispositifs permettent justement d'éviter le glissement du patient au sein de la structure. Les risques d'exposition de parties sensibles du corps du patient, telles que sa tête et plus particulièrement ses yeux, sont donc amoindris de manière significative.

Toutefois, ces dispositifs, comprenant une zone dite matelassée, sont conçus pour accueillir le patient préférentiellement en position foetale. Or, une position foetale implique que certaines zones du corps du patient ne sont pas exposées à la lumière émise par la source lumineuse. Ces dispositifs ne permettent donc pas d'avoir une exposition homogène sur l'intégralité du corps du patient. L'efficacité du traitement n'est donc pas maximale avec ces dispositifs.
On connait également de ce document des dispositifs souples, type couverture repliable autour du patient, et qui sont aptes à recevoir un pad à base de fibres optiques en vue d'un traitement par photothérapie. Toutefois, ce type de dispositif ne permet pas de réaliser un traitement à intensité importante sur de longue période par exemple, de 10 à 12 heures. En effet, la structure proposée ne présente aucun moyen d'évacuation de la chaleur générée par exposition lumineuse.

On connait également d'autres dispositifs souples tel que décrit dans le document WO 2013/068518 comprenant une couche à base de matériaux à changement de phase, qui permet d'effectuer des traitements sur des durées plus longues et à intensité lumineuse plus importante également. Bien qu'ayant amélioré le confort du patient d'un point de vue purement thermique grâce à une régulation automatique de la température, ce type de dispositif ne permet pas de maintenir le patient en position optimale. Compte tenu du volume d'illumination proposé par ce type de dispositif, des mouvements latéraux ne réduisent pas l'efficacité du traitement. En revanche, des mouvements de glissement du patient vers la partie basse du dispositif ne sont pas à exclure et constituent un inconvénient important. En effet, il est primordial d'éviter que la tête du patient, et plus précisément ces yeux, soient exposés aux rayons lumineux. Cependant, ces dispositifs ne permettent pas d'éviter ce scénario. En ce sens, ce type de dispositif est donc limité puisqu'il ne permet pas d'éviter des situations risquées d'exposition de parties sensibles du patient du fait de l'absence de limitation des mouvements du patient au cours du traitement.
A l'heure actuelle, ce dispositif souple de traitement de la jaunisse par photothérapie, ne permet pas de limiter les déplacements du patient uniquement dans le volume d'illumination selon au moins deux directions tout en évitant le confinement du patient en partie basse du dispositif, dans le but d'optimiser la surface d'exposition du patient.

WO 2009/139877 A1 décrit un dispositif textile de type lange pour l'emmaillotage du nourrisson.

On conçoit ainsi aisément le besoin d'un dispositif de traitement par photothérapie, souple et confortable, permettant l'exposition efficace du patient sur une plus longue durée.

### EXPOSE DE L'INVENTION

Le but de l'invention est donc de proposer un dispositif souple permettant d'obtenir un tel résultat.

A cet effet, l'invention a pour objet un dispositif textile monopatient pour le traitement par photothérapie d'un patient, comportant :
- une paroi dorsale destinée à recevoir le patient dans une position allongée ; la paroi dorsale comprenant une partie supérieure et une partie inférieure;
- une paroi ventrale rattachée à la partie inférieure de la paroi dorsale;
- deux parois latérales disposées de part et d'autre de la paroi dorsale et aptes à se replier sur celle-ci ;
les parois ventrale et latérale forment une fois repliées un volume pour le logement du patient comportant une ouverture pour le passage de la tête du patient au niveau de la partie supérieure de la paroi dorsale.
Conformément à l'invention, le dispositif textile monopatient se caractérise en ce que ladite paroi ventrale est apte à se replier sur ladite paroi dorsale, de sorte que les parois ventrale et dorsale présentent chacune une surface interne apte à diffuser la lumière dans ledit volume.

En d'autres termes, grâce à cette structure particulière, ce dispositif textile monopatient permet un positionnement efficace du patient, couché sur le dos, au niveau de la paroi dorsale. Ce dispositif textile monopatient possède une longueur suffisamment grande pour recevoir des nourrissons de tailles différentes. Une fois le patient correctement positionné, le praticien peut ainsi replier la partie ventrale dans le but de recouvrir l'intégralité du corps du patient.
On entend par intégralité du corps, l'ensemble du corps exception faite de la tête qui émerge au niveau de l'ouverture en partie supérieure du dispositif textile monopatient. Le patient se retrouve alors allongé en position « sandwich » entre la partie dorsale et la partie ventrale. Le dispositif textile monopatient est donc conçu pour positionner le patient en position allongée dans le but de diriger la lumière émise sur l'intégralité de son corps. Grâce à la présence d'une couche interne, permettant la transmission de lumière sur chacune des parois dorsale et ventrale, le patient est exposé intégralement. Avantageusement la surface interne diffuse au moins 75% de la lumière reçue.

Les parois latérales repliées permettent de définir le volume dans lequel le patient est logé. Avantageusement en pratique, la longueur des parois latérales est inférieure à la longueur de la paroi dorsale, de sorte que le volume comporte des ouvertures sur la partie inférieure de la paroi dorsale. Notamment, ces ouvertures permettent d'évacuer la chaleur générée par la lumière au cours du traitement et plus particulièrement lors d'une exposition de longue durée. Le dispositif textile monopatient autorise une certaine circulation d'air ce qui permet un meilleur confort du patient au cours du traitement et ce sans augmenter trop fortement les risques de fuite de lumière.

Selon un mode de réalisation, le dispositif textile monopatient comprend des moyens de maintien en position du patient qui sont solidaires de la partie dorsale.

Avantageusement en pratique, ces moyens de maintien sont formés d'au moins trois lanières cousues sur la partie dorsale. Deux lanières latérales permettant le maintien du bassin du patient sur la partie dorsale, préférentiellement au centre de celle-ci. Une troisième lanière permet de maintenir l'entre-jambe du patient et est destinée à être rattachée aux deux lanières transversales par un système d'adhésif ou analogue.

Ainsi, au cours du traitement, le patient est limité en termes de mouvements selon au moins deux directions, à savoir la direction transversale et la direction longitudinale. On évite donc tout glissement du patient vers la partie basse, et ce faisant, l'exposition de parties sensibles du patient telles que le visage, et plus précisément les yeux.

Selon un mode de réalisation préférentiel, les lanières protègent les parties génitales du patient et pour ce faire sont avantageusement réalisées à base d'un non tissé tel que le « spunbond » par exemple ou tous autres matériaux analogues remplissant cette même fonction.

Selon un mode de réalisation particulier, les moyens de maintien en position peuvent former une couche. En effet, les traitements sont généralement effectués sur de longues durées, la présence d'une couche intégrée au sein du dispositif trouve ainsi donc tout son sens.

En pratique, les parois dorsale et ventrale sont rattachées par une zone de pliure comportant au moins une pliure transverse permettant ainsi de rabattre la partie ventrale sur la zone du corps du patient destinée au traitement. La zone de pliure facilite le mouvement de repliement des deux parois et est préférentiellement situé à l'aplomb des pieds du patient.

Les parois dorsale et ventrale comprennent au moins deux couches superposées ; à savoir une couche formant la surface interne et une autre couche formant la surface externe du dispositif textile monopatient.

Avantageusement, la surface interne du dispositif textile monopatient est réalisée principalement d'un textile non tissé présentant avantageusement un contact doux au toucher, ce qui confère au dispositif textile des propriétés de confort.

Parallèlement, la surface externe peut être réalisée d'un textile ou d'un non-tissé atténuant les sorties de lumière dans le but de maximiser l'efficacité du traitement par photothérapie et de limiter les risques liés à l'exposition des yeux à la lumière intense.

En pratique, les parois dorsale et ventrale possèdent chacune un logement apte à recevoir respectivement une source émettrice de lumière. Ainsi, le patient étant directement au contact de ces deux parois, la lumière émise est alors principalement orientée en direction de son corps. Ce positionnement des sources émettrices de lumière permet au système de limiter l'émission de lumière vers l'extérieur du volume défini par le dispositif textile monopatient.

En outre, le dispositif permet de s'affranchir des risques liés à l'exposition de parties sensibles du corps du patient, outre les parties génitales, les yeux ne sont également pas exposés à la lumière au cours du traitement.

En pratique, la partie supérieure de la paroi dorsale comprend deux bretelles permettant de la solidariser avec la paroi ventrale une fois repliée. Ainsi, une fois ces bretelles repliées, le dispositif textile monopatient présente une ouverture limitée et contrôlée vers le haut ce qui permet d'éviter que la lumière émise au sein du volume ne se propage en direction des zones sensibles du corps du patient et plus particulièrement en direction des yeux.

En pratique, la paroi ventrale, les parois latérales et les bretelles possèdent respectivement des moyens de fixation permettant de les solidariser entre elles.

Les moyens de fixation sont préférentiellement de type velcros doux (tel que ceux utilisé pour les couches des nouveau-nés). Ils permettent notamment de maintenir efficacement le dispositif textile monopatient en position « repliée » lors du traitement, en prévenant de toute ouverture malgré les éventuels mouvements du patient. De plus, ils permettent, dans une position ouverte, que les parois latérales soient dépliées assurant ainsi une meilleure circulation de l'air.

Selon une variante, le dispositif textile monopatient comprend un dispositif de maintien des sources diffusant la lumière au sein des logements. Avantageusement, le dispositif de maintien est constitué de deux lanières solidaires qui, lorsqu'elles sont lacées entre elles permettent de maintenir les organes lumineux au sein des logements du dispositif textile monopatient. Ainsi, le positionnement de ces sources de lumière est assuré indépendamment des mouvements du patient.

En pratique, une installation pour le traitement par photothérapie d'un patient comprend au moins :
- un dispositif textile monopatient tel que défini précédemment ;
- une source de lumière ;
- au moins un organe lumineux réalisé en fibres optiques ; les fibres optiques étant raccordées à la source de lumière par un câble de fibres optiques muni d'un connecteur optique et le au moins un organe lumineux étant apte à être intégré dans au moins un des logements du dispositif textile monopatient.

Avantageusement, les fibres optiques émettent à une lumière longueur d'onde comprise entre 400 nm et 520 nm, préférentiellement comprise entre 430 et 490 nm (longueur d'onde correspondant au maximum d'absorption de la bilirubine).

### DESCRIPTION SOMMAIRE DES FIGURES

L'invention sera mieux comprise à la lecture de la description qui va suivre, donnée uniquement à titre d'exemple, nullement limitative, et réalisée en relation avec les dessins annexés dans lesquels :
- la figure 1 est une vue schématique de dessus d'un dispositif textile monopatient de traitement par photothérapie, déplié, selon un mode de réalisation préférentiel de l'invention ;
- la figure 2 vue schématique de dessous du dispositif textile monopatient de la figure 1 ;
- la figure 3 est une vue schématique du dispositif textile monopatient de la figure 1 représentant le cas où lesdits organes lumineux sont insérés dans lesdits logements et ladite paroi ventrale est repliée ;
- la figure 4 est une vue schématique de dessus du dispositif de la figure 1, représentant le cas où ledit moyen de maintien est replié ;
- la figure 5 est une vue schématique de dessus du dispositif textile monopatient, de la figure 1, en position repliée, au sein duquel un patient est couché sur le dos ;
- la figure 6 est une vue schématique d'une installation de traitement par photothérapie intégrant ledit dispositif textile monopatient de la figure 1.

### MANIERE DE REALISER L'INVENTION

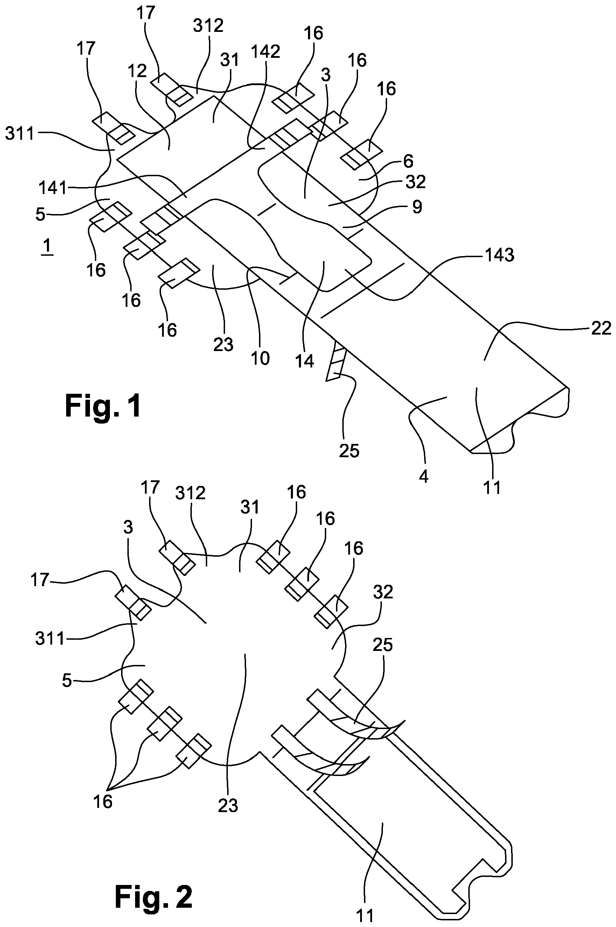

Sur la figure 1, est représenté un dispositif textile monopatient **1** pour traitement par photothérapie comprend une paroi dorsale **3** présentant une partie supérieure **31** et une partie inférieure **32.**

Le dispositif textile monopatient **1** présente également un partie dite ventrale **4** qui est rattachée à la partie dorsale **3** au niveau de sa partie inférieure **32.**

La paroi ventrale **4** est rattachée à la paroi dorsale **3** au niveau d'une zone dite de pliure **9** présentant une pliure transverse **10.** Avantageusement en pratique, la paroi ventrale **4** est apte à se replier sur la paroi dorsale **3.**

Les parties dorsale **3** et ventrale **4** sont composées de deux couches **22,23.** La couche **22** forme la surface interne du dispositif textile monopatient **1,** alors que la couche **23** forme, quant à elle, la surface externe du dispositif textile monopatient **1.**

La couche **22** est avantageusement réalisée d'un textile non tissé à base de viscose et/ou coton. Par exemple, la couche **22** est réalisée à base d'un matériau de type « spunlace » ou analogues.

La couche **22** est apte à laisser passer la lumière au cours du traitement par photothérapie. L'objectif recherché est de minimiser au maximum l'absorption ou réflexion de la lumière au niveau de la couche **22.** Pour ce faire, la couche **22** présente avantageusement une masse surfacique maximale de 60 g/m². Dans ce cas, la couche **22** est suffisamment fine pour laisser passer au minimum 75% de lumière, et de ce fait, augmenter l'efficacité du traitement du patient **2.**

Au cours de traitement de longue durée (jusqu'à 24 heures en continu) à forte intensité lumineuse, le patient se trouve dans un environnement dont les conditions de température peuvent être relativement élevées provoquant une sudation du patient. Le dispositif textile monopatient **1** présente donc des propriétés d'absorption de l'eau suffisantes pour assurer le confort du patient. La couche **22** présente une absorption à l'eau supérieure à 400%, avantageusement supérieure à 800 %.

Tel que représenté sur la figure 2, la couche **23** formant la surface externe des parois dorsale **3** et ventrale **4** est, quant à elle, réalisée d'un textile non tissé à base principalement de polypropylène. Le matériau formant la couche **23** est avantageusement de type « spunbond » ou autres matériaux analogues.

L'objectif étant de concentrer un maximum de lumière en direction du patient au cours du traitement, la couche **23** présente ainsi des propriétés d'opacité permettant d'atteindre un tel résultat.

Par ailleurs, la couche **23,** étant exposée au milieu extérieur, n'est donc pas directement au contact du patient. Elle présente une capacité d'absorption à l'eau inférieure à la couche **22,** avantageusement comprise entre 100 et 150 %.
principalement de polypropylène. Le matériau formant la couche **23** est avantageusement de type « spunbond » ou autres matériaux analogues.

L'objectif étant de concentrer un maximum de lumière en direction du patient au cours du traitement, la couche **23** présente ainsi des propriétés d'opacité permettant d'atteindre un tel résultat.

Par ailleurs, la couche **23,** étant exposée au milieu extérieur, n'est donc pas directement au contact du patient. Elle présente une capacité d'absorption à l'eau inférieure à la couche **22,** avantageusement comprise entre 100 et 150 %.

La paroi dorsale **3** est destinée à accueillir le patient au cours du traitement. Dans le but de remédier à un risque majeur du traitement qui est celui de l'exposition des parties sensibles du corps du patient telles que les yeux, le dispositif textile monopatient **1** comprend avantageusement un moyen de maintien **14** en position du patient qui est solidaire de la partie dorsale **3.**

Tel que représenté sur la figure 1, ce moyen de maintien **14** comprend trois lanières **141,142,143.**

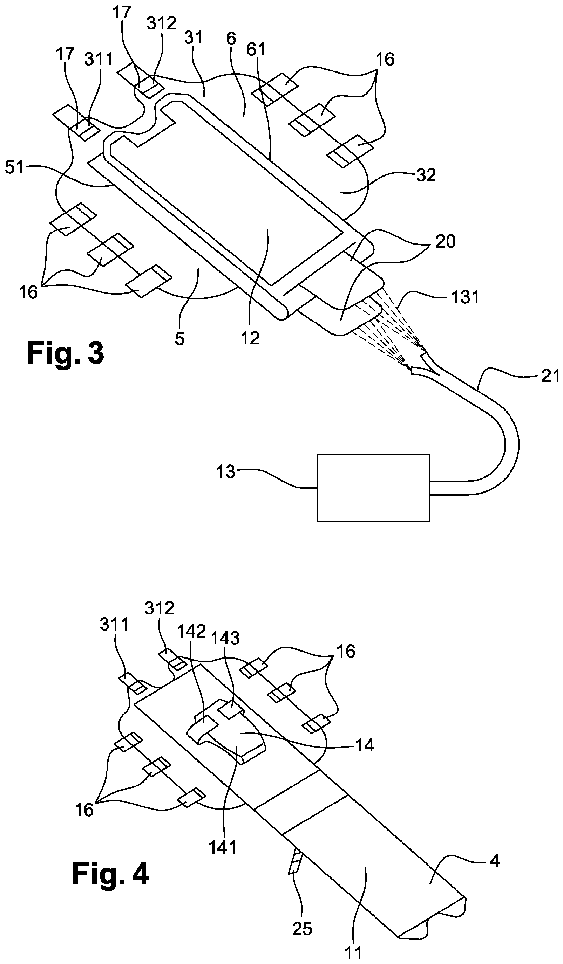

Tel que représenté sur la figure 4, deux lanières latérales **142,143** permettent un maintien du bassin du patient, limitant ainsi les mouvements latéraux au sein du dispositif textile monopatient **1.** Une troisième lanière **141,** apte à se replier, permet le maintien de l'entrejambes du patient, évitant ainsi tout glissement vers la partie basse du dispositif textile monopatient **1.**

Avantageusement en pratique, les trois lanières **141,142,143** sont cousues sur la partie dorsale **3** et réalisées en un matériau identique à celui de la surface externe **23.**

Selon un mode de réalisation particulier, le moyen de maintien forme une couche pour nouveau-né composée par les trois lanières.

Tel que représenté sur la figure 3, les parois dorsale **3** et ventrale **4** présentent chacune un logement **11,12** aptes à recevoir respectivement des organes lumineux **20** de fibres

En outre, les parois latérales **5,6** sont aptes à se replier sur la paroi dorsale **3.** Elles présentent donc chacune une zone de pliure **51** et **61,** préférentiellement longitudinale par rapport à la paroi dorsale **3.**

Le dispositif textile monopatient **1** présente, en outre, deux bretelles **311,312** de solidarisation de la partie supérieure **31** de la paroi dorsale **3** avec la paroi ventrale **4.** Ces bretelles **311,312** sont préférentiellement réalisées d'un matériau similaire à celui de la couche externe **23.**

Tel que représenté sur la figure 1, les parois latérales **5,6,** les bretelles **311,312** ainsi que la paroi ventrale **4** sont munies de moyens de fixation **16,17** avantageusement de type velcro doux ou autres analogues.

Ainsi une fois repliées, un volume **7** est alors formé au sein du dispositif textile monopatient **1** définissant le volume **7** pour le logement du patient.

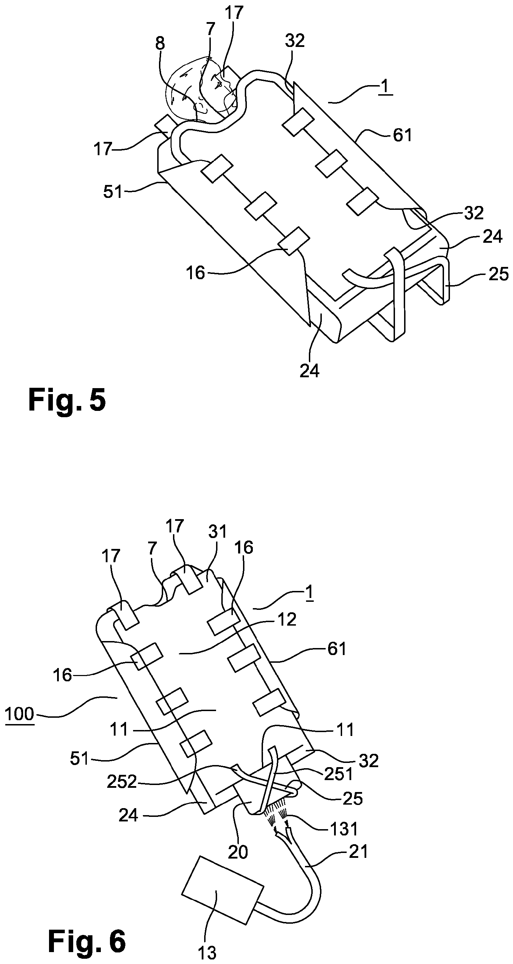

Tel que représenté sur la figure 5, la tête du patient **2** émerge du dispositif textile monopatient **1** au niveau d'une ouverture **8.**

La longueur des parois latérales **5,6** est inférieure à la longueur de la paroi dorsale **3** de sorte que le volume **7** comporte des ouvertures **24** sur la partie inférieure **32** de la paroi dorsale **3.**

Avantageusement en pratique, la longueur des parois latérales **5,6** est comprise entre 350 et 400 mm, préférentiellement de 360 mm.

En outre, la longueur de la paroi dorsale **3** est comprise entre 400 et 500 mm, préférentiellement égale à 460 mm.

Chacune des ouvertures **24** évacue de la chaleur de sorte que la température au sein du dispositif textile monopatient **1** puisse être ajustée par le personnel soignant : en position repliée les calories sont conservées au sein du dispositif **1,** les ouvertures latérales **24** servent à abaisser la température si nécessaire.

Le dispositif textile monopatient **1** présente, en plus, un dispositif de fixation **25** des organes lumineux **20** au sein des logements **11,12** composé de deux lanières **251,252** destinées à resserrer la partie inférieure **32** du dispositif **1.** Les lanières **251,252** sont préférentiellement réalisées à base de spunbond.

Il a été décrit un mode particulier de l'invention. Cependant, de nombreuses variantes peuvent y être apportées, comme celles-ci-dessous, prises seules ou en combinaison.

Il a été décrit un dispositif textile monopatient présentant des parois latérales de part et d'autre de la partie dorsale. En variante, les parois latérales peuvent être prévues de part et d'autre de la partie ventrale destinée à être repliée sur la partie dorsale.

Il a été décrit un système de lanières comme moyen de maintien du patient en position. Toutefois, tout autre type de système de maintien peut être intégré au sein du dispositif textile monopatient selon l'invention.

Il a été décrit une zone de pliure comprenant une seule pliure transverse. Selon un autre mode de réalisation, la zone de pliure présente au moins deux pliures transverses.

Il a été décrit des moyens de fixations de type velcro doux (hoop&loop system). Cependant, tous autres types de moyens de fixation connus peuvent être intégrer au dispositif textile monopatient de l'invention.

Tel que représenté sur la figure 6, un dispositif **100** pour le traitement par photothérapie d'un patient **2,** comprend au moins :
- un dispositif textile monopatient **1** tel que décrit précédemment ;
- une source de lumière **13** ;
- au moins un organe lumineux **20** réalisé en fibres optiques **131** ; lesdites fibres optiques **131** étant raccordées à la source de lumière **13** par un câble **21** muni d'un connecteur optique et ledit au moins organe lumineux **20** étant apte à être intégré dans au moins un des logements **11,12** dudit dispositif textile monopatient **1.**

La source de lumière **13** présente un éclairement spectral pouvant varier de 30 µW/cm.²nm à 100 µW/cm.²nm pour une longueur d'onde comprise entre 400 et 520 nm.

Le pic de longueur d'onde de la lumière émise par la source **13** est compris entre 400nm et 520 nm ce qui correspond à la couleur bleue du spectre de la lumière visible et au pic d'absorption de la bilirubine.

Les fibres optiques **131** sont préférentiellement réalisées à base d'un matériau polymère afin de conduire la lumière émise par la source d'éclairage **13** efficacement jusqu'aux organes lumineux **20.**

Par exemple, les fibres optiques présentent un coeur enrobé au moins partiellement par une gaine et peuvent être réalisées à base de polyméthylméthacrylate (coeur) et de polymère fluoré (gaine).

Les fibres optiques **131** font éventuellement l'objet d'un traitement latéral sur leur longueur qui est adapté à l'application. Avantageusement en pratique, les fibres **131** émettent de manière unidirectionnelle vers le patient **2.**

En outre, les fibres **131** sont avantageusement disposées parallèlement au sein des organes lumineux **20.**

Le câble **21** est préférentiellement réalisé d'un matériau opaque, à base de polyoléfine recouvert d'un film polyuréthane de couleur blanche.

Avantageusement en pratique, les organes lumineux **20** ont une géométrie rectangulaire et ont une longueur comprise entre 300 et 600 mm et une largeur comprise entre 200 et 500 mm. Les dimensions des organes lumineux **20** sont légèrement inférieures aux dimensions des logements **11,12** qui les accueillent.

De par sa composition et sa simplicité de conception, le dispositif textile monopatient **1** est avantageusement destiné à un usage unique.

Toutefois, le dispositif textile peut également subir plusieurs lavages afin d'être réutilisé.

## Revendications

1. Dispositif textile monopatient (1) pour le traitement par photothérapie d'un patient (2), comportant ;
• une paroi dorsale (3) destinée à recevoir ledit patient (2) dans une position allongée, ladite paroi dorsale (3) comprenant une partie supérieure (31) et une partie inférieure (32) ;
• une paroi ventrale (4) rattachée à la partie inférieure (32) de ladite paroi dorsale (3);
• deux parois latérales (5,6) disposées de part et d'autre de ladite paroi dorsale (3) et aptes à se replier sur celle-ci ;
lesdites parois ventrale (4) et latérales (5,6) formant une fois repliées un volume (7) pour le logement du patient (2) comportant une ouverture (8) pour le passage de la tête du patient (2) au niveau de la partie supérieure (31) de la paroi dorsale (3) ; et
ladite paroi ventrale (4) est apte à se replier sur ladite paroi dorsale (3) de sorte que lesdites parois ventrale (4) et dorsale (3) présentent chacune une surface interne (22) apte à diffuser de la lumière dans ledit volume (7)
***caractérisé* en ce qu'**il comprend en outre :
un moyen (14) de maintien en position du patient, ledit moyen de maintien étant solidaire de ladite paroi dorsale (3) et étant formé d'au moins trois lanières (141, 142, 143) parmi lesquelles deux lanières latérales (142, 143) destinées au maintien du bassin du patient sur la paroi dorsale et une troisième lanière (141) destinée au maintien de l'entrejambes du patient, ladite troisième lanière (141) étant en outre destinée à être rattachée aux deux lanières transversales (142, 143).

2. Dispositif selon la revendication 1, ***caractérisé* en ce que** la longueur des parois latérales (5,6) est inférieure à la longueur de la paroi dorsale (3) de sorte que le volume (7) comporte des ouvertures (24) sur la partie inférieure (32) de la paroi dorsale (3).

3. Dispositif selon l'une des revendications précédentes, ***caractérisé* en ce que** lesdites parois dorsale (3) et ventrale (4) comprennent au moins deux couches (22,23) superposées ; une première couche (22) formant la surface interne et une deuxième couche (23) formant la surface externe dudit dispositif textile monopatient (1).

4. Dispositif selon l'une des revendications précédentes, ***caractérisé* en ce que** ladite surface interne (22) diffuse au moins 75% de la lumière reçue.

5. Dispositif selon l'une des revendications précédentes, ***caractérisé* en ce que** lesdites parois dorsale (3) et ventrale (4) possèdent chacune un logement (11,12) aptes à recevoir respectivement une source diffusant la lumière (13,14).

6. Dispositif selon l'une des revendications précédentes, ***caractérisé* en ce que** ladite partie supérieure (31) de la paroi dorsale (3) comprend deux bretelles (311,312) de solidarisation de la partie supérieure (31) de la paroi dorsale (3) avec la paroi ventrale (4) une fois repliée.

7. Dispositif selon la revendication 6, ***caractérisé* en ce que** chacune des parois ventrale (4), latérales (5) et lesdites brettelles (311,312) possèdent respectivement des moyens de fixations (15,16,17) entre elles.

8. Dispositif selon la revendication 5, ***caractérisé* en ce qu'**il comprend un dispositif de maintien (18) desdites sources diffusant la lumière (13,14) au sein desdits logements (11,12).

9. Installation (100) pour le traitement de photothérapie d'un patient (2) comprenant au moins :
- un dispositif textile monopatient (1) selon l'une des revendications précédentes ;
- une source de lumière (13) ;
- au moins un organe lumineux (20) réalisé en fibres optiques (131) ; lesdites fibres optiques (131) étant raccordées à la source de lumière (13) par un câble (21) de fibres optiques (131) muni d'un connecteur optique (21) et ledit au moins organe lumineux (20) étant apte à être intégré dans au moins un des logements (11,12) dudit dispositif textile monopatient (1).

10. Installation (100) selon la revendication 11, ***caractérisé* en ce que** la source de lumière émet une longueur d'onde comprise entre 400 nm et 520 nm.

## Patentansprüche

1. Textile Einzelpatient-Vorrichtung (1) zur Fototherapie für einen Patienten (2), umfassend:
- eine Rückwand (3), die dazu bestimmt ist, den Patienten in einer ausgestreckten Lage aufzunehmen, wobei diese Rückwand (3) einen oberen Abschnitt (31) und einen unteren Abschnitt (32) aufweist;
- eine Bauchwand (4), die mit dem unteren Abschnitt (32) dieser Rückwand (3) verbunden ist;
- zwei Seitenwände (5, 6), die auf jeder Seite der Rückwand (3) angeordnet sind und die auf die Rückwand gefaltet werden können;
wobei diese Bauchwand (4) und die Seitenwände (5, 6) einen Raum (7) zur Unterbringung des Patienten (2) mit einer Öffnung (8) zur Durchführung des Kopfes des Patienten in Höhe des oberen Abschnitts (31) der Rückwand (3) bilden, sobald sie aufgefaltet sind, und
wobei diese Bauchwand (4) über diese Rückwand (3) gefaltet werden kann, so dass die Bauch- (4) und die Rückwand (3) jeweils eine Innenfläche aufweisen (22), die Licht innerhalb dieses Raums (7) verteilen kann.
***dadurch gekennzeichnet,* dass** sie außerdem:
eine Vorrichtung (14) umfasst, um den Patienten in Position zu halten, wobei diese Haltevorrichtung fest mit dieser Rückwand (3) verbunden ist und aus mindestens drei Riemen (141, 142, 143) besteht, unter denen die beiden seitlichen Riemen (142, 143) dazu bestimmt sind, das Becken des Patient an der Rückwand zu halten und ein dritter Riemen (141) dazu dient, den Schrittbereich dieses Patienten zu halten, wobei dieser dritte Riemen (141) außerdem dazu bestimmt ist, mit den beiden Querriemen (142, 143) verbunden zu werden.

2. Vorrichtung gemäß Anspruch 1, ***dadurch gekennzeichnet,* dass** die Länge der Seitenwände (5, 6) kürzer ist, als die Länge der Rückwand (3), so dass der Raum (7) Öffnungen (24) im unteren Abschnitt (32) der Rückwand (3) enthält.

3. Vorrichtung gemäß einem der vorangehenden Ansprüche, ***dadurch gekennzeichnet* dass** diese Rück- (3) und Bauchwand (4) aus mindestens zwei übereinander geschichten Schichten (22, 23) bestehen, wobei eine erste Schicht (22) die Innenfläche bildet und eine zweite Schicht (23) die Außenfläche dieser textilen Einpatient-Vorrichtung (1) bildet.

4. Vorrichtung gemäß einem der vorangehenden Ansprüche, ***dadurch gekennzeichnet,* dass** diese Innenfläche (22) mindestens 75 % des empfangenen Lichtes verteilt.

5. Vorrichtung gemäß einem der vorangehenden Ansprüche, ***dadurch gekennzeichnet,* dass** diese Rück- (3) und Bauchwand (4) jeweils eine Vorrichtung (11, 12) zur Aufnahme jeweils einer das Licht verbreitenden Quelle (13, 14) enthalten.

6. Vorrichtung gemäß einem der vorangehenden Ansprüche, ***dadurch gekennzeichnet,* dass** dieser obere Abschnitt (31) der Rückwand (3) zwei Gurte (311, 312) zur Befestigung des oberen Abschnitts (31) der Rückwand (3) an der Bauchwand (4) enthält, sobald sie aufgefaltet ist.

7. Vorrichtung gemäß Anspruch 6, ***dadurch gekennzeichnet,* dass** sowohl die Bauchwand (4) wie die Seitenwände und diese Gurte (311, 312) jeweils über Vorrichtungen verfügen, durch die sie miteinander verbunden werden können (15, 16, 17).

8. Vorrichtung gemäß Anspruch 1, ***dadurch gekennzeichnet,* dass** sie eine Vorrichtung zum Halten dieser das Licht verteilenden Quellen (13, 14) innerhalb dieser Unterbringungsvorrichtungen (11, 12) enthält.

9. Gerät (100) zur Fototherapie für einen Patienten (2), umfassend mindestens:
- eine textile Einzelpatient-Vorrichtung (1) nach einem der vorangehenden Ansprüche;
- eine Lichtquelle (13);
- mindestens ein Leuchtmittel (20) aus optischen Fasern (131), wobei diese optischen Fasern (131) mit der Lichtquelle (13) über ein Kabel (21) aus optischen Fasern (131) verbunden sind, das mit einem optischen Steckverbinder (21) ausgerüstet ist und wobei dieses mindestens ein Leuchtmittel (20) in mindestens einer der Unterbringungsvorrichtungen (11, 12) dieser textilen Einpatient-Vorrichtung (1) untergebracht werden kann.

10. Gerät (100) gemäß Anspruch 11, ***dadurch gekennzeichnet,* dass** die Lichtquelle eine Wellenlänge zwischen 400 nm und 520 nm aussendet.

## Claims

1. A single-patient textile device (1) for providing phototherapy treatment to a patient (2), including:
• a back wall (3) intended to accommodate said patient (2) in a supine position, said back wall (3) comprising an upper portion (31) and a lower portion (32);
• a front wall (4) attached to the lower portion (32) of said back wall (3);
• two lateral walls (5, 6) which are arranged on either side of said back wall (3) and can be folded onto said wall;
once folded, said front wall (4) and lateral walls (5, 6) forming a volume (7) to house the patient (2), including an opening (8) to enable the passing of the patient's (2) head through the upper portion (31) of the back wall (3); and
said front wall (4) can be folded onto said back wall (3), such that said front wall (4) and said back wall (3) each has an inner surface (22) that can distribute the light within said volume (7);
***characterized* in that** it further comprises:
a position-holding mean (14) for holding the patient in place, said holding mean being integral to said back wall (3) and comprising at least three straps (141, 142, 143), among which two side straps (142, 143) for holding the patient's pelvis onto the back wall (3) and a third strap (141) for holding the crotch of the patient, said third strap (141) being intended to be attached to the two side straps (142, 143).

2. The device according to claim 1, ***characterized* in that** the length of the lateral walls (5,6) is less than the length of the back wall (3), such that the volume (7) includes openings (24) into the lower portion (32) of the back wall (3).

3. The device according to any of the preceding claims, ***characterized* in that** said back wall (3) and said front wall (4) comprise at least two superimposed layers (22, 23); a first layer (22) that forms the inner surface and a second layer (23) that forms the outer surface of said single-patient textile device (1).

4. The device according to any of the preceding claims, ***characterized* in that** said inner surface (22) disseminates at least 75% of the received light.

5. The device according to any of the preceding claims, ***characterized* in that** said back wall (3) and said front wall (4) each has a housing (11, 12) that can accommodate a light emitting source (13, 14), respectively.

6. The device according to any of the preceding claims, ***characterized* in that** said upper portion (31) of the back wall (3) comprises two braces (311, 312) to secure the upper portion (31) of the back wall (3) to the front wall (4) once the latter has been folded.

7. The device according to claim 6, ***characterized* in that** the front wall (4), the lateral walls (5) and said braces (311, 312) each has fastening means (15, 16, 17) to secure them to one another.

8. The device according to claim 5, ***characterized* in that** it comprises a device (18) for holding said light emitting sources (13, 14) within said housings (11, 12).

9. A unit (100) for providing phototherapy treatment to a patient (2) comprising at least:
- one single-patient textile device (1) according to one of the preceding claims;
- one light source (13);
- at least one luminous member (20) made up of optical fibers (131); said optical fibers (131) being connected to the light source (13) by the cable (21) made up of optical fibers (131) equipped with an optical connector (21) and said at least one luminous member (20) being suitable to be integrated into at least one of the housings (11, 12) of said single-patient textile device (1).

10. The unit (100) according to claim 9, ***characterized* in that** the light source emits a wavelength between 400 nm and 520 nm.
